# EUROPEAN PATENT SPECIFICATION

(11) **EP 4 176 106 B1**
(45) Date of publication and mention of the grant of the patent: **09.10.2024**
(21) Application number: 21735739.1
(22) Date of filing: 05.07.2021
(51) Int. Cl.: C25D 5/44, C25D 11/02, C25D 11/04, C25D 11/12, C25D 11/20, C25D 11/24, A61L 31/02, A61L 31/08, C23C 28/00, C25D 3/38

(54) **A METHOD FOR MANUFACTURING COPPER FILM ON POROUS ALUMINUM OXIDE (PAO) ON AN ALUMINUM ALLOY SUBSTRATE**
VERFAHREN ZUR HERSTELLUNG EINES KUPFERFILMS AUF PORÖSEM ALUMINIUMOXID (PAO) AUF EINEM SUBSTRAT AUS EINER ALUMINIUMLEGIERUNG
PROCÉDÉ DE FABRICATION D'UN FILM DE CUIVRE SUR DE L'OXYDE D'ALUMINIUM POREUX (PAO) SUR UN SUBSTRAT EN ALLIAGE D'ALUMINIUM

(30) Priority: 06.07.2020 EP 20184136
(43) Date of publication of application: 10.05.2023
(73) Proprietor: Mion Technology ApS, 7080 Børkop (DK)
(72) Inventor: MORGEN, Per, 5000 Odense C (DK)
(74) Representative: Plougmann Vingtoft a/s
(86) International application number: PCT/EP2021/068507
(87) International publication number: WO 2022/008439

(56) References cited:
- CH-A2- 709 962
- CN-A- 106 637 310
- US-A- 3 468 765
- US-A- 5 132 003
- US-A1- 2019 279 792
- T. I. DEVYATKINA ET AL: "Anodic oxidation of complex shaped items of aluminum and aluminum alloys with subsequent electrodeposition of copper coatings", RUSSIAN JOURNAL OF APPLIED CHEMISTRY, vol. 87, no. 1, 1 January 2014 (2014-01-01), Moscow, pages 54 - 60, XP055761550, ISSN: 1070-4272, DOI: 10.1134/S107042721401008X
- D KANAKARN ET AL: "PLATING ON ANODISED ALUMINIUM", BULLETIN OF ELECTROCHEMISTRY, 1 January 1986 (1986-01-01), pages 273 - 275, XP055761551, Retrieved from the Internet <URL:http://cecri.csircentral.net/2116/1/40-1986.pdf> [retrieved on 20201218]
- M W LOSEY ET AL: "Journal of the Electrochemical Society Nucleation and Adhesion of Electrodeposited Copper on Anodized Thin- Film Aluminum for LIGA Microfabrication Nucleation and Adhesion of Electrodeposited Copper on Anodized Thin-Film Aluminum for LIGA Microfabrication Through", J. ELECTROCHEM. SOC, 1 January 2006 (2006-01-01), XP055761552, Retrieved from the Internet <URL:https://iopscience.iop.org/article/10.1149/1.2354453/pdf>
- MONIKA NEHRA ET AL: "Highly Ordered and Crystalline Cu Nanowires in Anodic Aluminum Oxide Membranes for Biomedical Applications", PHYSICA STATUS SOLIDI. A: APPLICATIONS AND MATERIALS SCIENCE, vol. 217, no. 13, 29 January 2020 (2020-01-29), DE, pages 1900842, XP055761549, ISSN: 1862-6300, DOI: 10.1002/pssa.201900842
- LIXIA YING ET AL: "Study on Direct Current Electro-Deposition of Copper Nanowires in Anodic Alumina Membrane Pores", COATINGS, vol. 9, no. 6, 11 June 2019 (2019-06-11), pages 378, XP055761795, DOI: 10.3390/coatings9060378
- CHUNG C-K ET AL: "Fabrication of copper nanowires using overpotential electrodeposition and anodic aluminium oxide template", MICRO & NANO LETTERS, THE INSTITUTION OF ENGINEERING AND TECHNOLOGY, MICHAEL FARADAY HOUSE, SIX HILLS WAY, STEVENAGE, HERTS. SG1 2AY, UK, vol. 8, no. 10, 1 October 2013 (2013-10-01), pages 579 - 581, XP006047888, DOI: 10.1049/MNL.2013.0269

## Description

### FIELD OF THE INVENTION

The present invention relates to a method for manufacturing a copper film on a porous aluminum oxide (PAO) film, or interlayer, on an aluminum alloy substrate. The invention also relates to a corresponding object with a copper film on a porous aluminum oxide (PAO) film on an aluminum alloy substrate, for example the object may be part of a building, or the object may be part of health equipment, or a medical device, etc. The object may alternatively be part of a current conductor, such as high-power applications.

### BACKGROUND OF THE INVENTION

Copper (chemical element: Cu) in various pure forms and as an alloying element has an exceptionally high activity towards killing bacteria and viruses lodging on its surfaces compared to other metals and organic or inorganic compounds. For some applications, silver may also have a relatively high activity towards killing bacteria and viruses, but copper is commonly believed to be a more effective metal than silver.

This has recently, and especially under the current virus pandemic (2020), become an important argument for using copper and its alloys, such as brass, in the manufacture of items for use in the health and hygiene sectors. However, in some hospitals, the use of pure or plated copper items is not accepted for aesthetic reasons (for example finger prints remains visible for some time on the surface), in contrast to the trend in the US for example where copper items are often accepted.

Aluminum (chemical element: Al, America English spelling is aluminum, British English spelling is aluminium) in various forms and alloys is in high demand as a replacement of copper in various sectors, where possible, due to a current price advantage of a factor of about 3-5 in favor of aluminum.

Applications, where both metals i.e. copper and aluminum, for example in the electricity sector, are used simultaneously, or in contact, are very rare due to their difference in electronegativity. Thus, aluminum is normally the more easily corroded partner of joints between these two metals in humid environments. Thus, the combination of copper and aluminum may not be stable from an electrochemical point of view, known as galvanic corrosion (also known as 'bimetallic corrosion' or 'dissimilar metal corrosion') of this particular metal pair.

EP 2 754 734 aims at providing an anti-virus aluminum member capable of minimizing secondary infections by deactivating viruses in a short period of time even when viruses adhere thereto, regardless of whether a viral envelope is present, and useful for application in door knobs, handrails, air-conditioner fins or the like. It is proposed that to use an anti-virus aluminum member capable of deactivating viruses that adhere thereto is characterized in that an anti-virus inorganic compound, such as copper or silver or alloys thereof, is present in the pores of an anodized membrane provided with multiple pores and obtained by anodizing aluminum or an aluminum alloy. However, an electrochemical stable combination of copper on aluminum is not disclosed or in any way suggested.

US 3,468,765 discloses a method of applying copper plating to an aluminum substrate comprising: cleaning the aluminum surface, anodizing the clean surface in a phosphoric acid solution, washing with water and transferring the wet aluminum substrate to an aqueous acid-copper plating tank containing a copper plating solution. However, single anodizing in a phosphoric acid solution leads to disordered pores with large diameters weakens the durability of the copper plating.

T. I. Devyatkina et al: "Anodic oxidation of complex shaped items of aluminum and aluminum alloys with subsequent electrodeposition of copper coatings", from Russian Journal of applied Chemistry, vol. 87, no. 1, 1 January 2014, pages 54-60, discloses a method of anodization of aluminum and aluminum alloys, which can be applied for subsequent plating of highly adherent copper coating instead of the known zincate treatment with additional annealing. The process comprises anodization of the aluminum alloy substrate in an acid mixture of composition 15 vol % H₂SO₄ and 15 vol % H₃PO₄ in the presence of various additives creating an oxide film. The additives performs an electrochemical activation to increase porosity of oxide layers during anodization to increase coating deposition during copper plating performed after the anodizing and washing in a standard sulfuric acid electrolyte allowing electroplating of a monolayer copper coating onto any aluminum alloys. However, using additives may lead to an inhomogeneous anodization and it is doubtful the pores have been filled weakening the durability of the copper coating.

US 5,132,003 discloses a process for surface treatment in which the surface of aluminum or its alloy can be put in a desired color and the improved wear resistance and corrosion resistance can be obtained. The process is characterized in that the process comprises the steps of: forming anodic oxidation coatings by conventional method on the surface of the aluminum or its alloy, thereafter applying an alternating voltage of 10 V ~ 30 V within a sulfate solution or nitrate solution of a desired metal to a member on which said anodic oxidation coatings was formed by the above step, whereby said metal is electrolytically impregnated into said anodic oxidation coatings. However, the method does not include preparing the aluminum surface by anodizing and therefore the pores are formed irregular and the coating may not be durable.

M.W. Losey et al, "Nucleation and Adhesion of Electrodeposited Copper on Anodized Thin-Film Aluminum for LlGA Microfabrication", 2006 J. Electrochem. Soc. 153 D177 discloses a process for manufacturing a thin and stable copper film on a porous aluminum oxide (PAO) film on an aluminum alloy sputtered film, the electrodepositing step comprising the use of direct current (DC) under vigorous agitation.

Hence, an improved method for manufacturing an object of an aluminum alloy with anti-virus/bacterial properties would be advantageous, and in particular a more efficient and/or reliable improved method of manufacturing such an object would be advantageous in various applications.

### OBJECT OF THE INVENTION

It is an object of the present invention to provide an alternative to the prior art.

In particular, it is an object of the present invention to provide a method for manufacturing an improved object of an aluminum alloy that solves the above mentioned problems of the prior art with electrochemical stability and/or adhesion.

### SUMMARY OF THE INVENTION

Thus, the above described object and several other objects are intended to be obtained in a first aspect of the invention by providing a process for manufacturing a thin and stable copper film on a porous aluminum oxide (PAO) film on an aluminum alloy substrate, the process comprising:
- anodization of the aluminum alloy substrate resulting in a regularly ordered PAO film; the PAO film and the remaining aluminum alloy thereby forming a PAO/aluminum substrate,
- optionally etching said PAO/aluminum substrate for further opening of the top of the pores in the surface of said PAO film after said anodization,
   and
- electrodepositing copper on said PAO/aluminum substrate, the electrodepositing comprises the use of direct current (DC), preferably in the interval of 5-20 V, more preferably 10-15 V, so as to deposit metallic copper both:
   1) in the open pores of the regularly ordered PAO film,
      and simultaneously or subsequently,
   2) on the top of the regularly ordered PAO film,

so as to form a copper film on top of the PAO film,
wherein said anodization is performed so as to improve the adhesion and electrochemical corrosion stability of the later electrodeposited copper on said PAO/aluminum substrate.

The invention is particularly, but not exclusively, advantageous for obtaining a suitable way to deposit copper on aluminum without the problems of galvanic or contact corrosion i.e. electrochemical stability. This could then be used in the manufacturing of different kinds of lighter elements of goods, based on aluminum, with anticorrosive and antimicrobial/viral surface functionalization from the properties of a thin added copper film coverage of the aluminum elements. With suitable processing steps, resulting in a surface of porous alumina and pores filled with copper, the surface also becomes self-cleaning due to its strongly hydrophobic nature.

An important aspect of this invention was found by following a procedure for anodization of aluminum, to first produce a strong anticorrosive oxide coverage of the metal. With suitable electrolytic procedures this oxide becomes porous in a self-organizing process unique to only a few metals with a relatively well-ordered structure of two different types of oxides on the surface. The structural or regular order, i.e. depth/thickness and pore diameters and pore ordering, is controllable by the proper choice of anodization procedure.

Typically, this porous structure in aluminum is attractive by easily lending itself to coloring of the surface with nanoparticles of colorants that enter the pores. The treatment is then finished by sealing the surface in a procedure acting to dissolve and close the top part of the pore structure. This leaves a smooth surface that is corrosion and smudging resistant, and thus easy to clean.

It has been thoroughly demonstrated by the present inventor how proper procedures, with high purity aluminum sheets and commercial aluminum alloys, may form a regular pore structure, which is then readily subjected to electrochemical treatments with copper ions to fill the pores and to grow a film on the surface consisting of copper. The structure of this film depends on the procedure used for the filling of the pores and on the subsequent growth mechanisms of the copper on top of the pores. For both cases, i.e. filling of the pores with copper metal or growth of a copper film on top of the surface with contact to the filled pores, the resulting surfaces are highly antimicrobial/viral.

In the case of filling of the pores, the surface shows the normal color and reflectivity as for pure anodized aluminum, without coloring. In the case of growth of the copper films with the filled pores below these films the sample may be mechanically polished to a smooth finish with the usual color of copper, but without the tendency of pure copper of copper plated surfaces without lacquered coverage to smudge or to corrode. The surface with pores filled with copper is highly hydrophobic, which acts as a self-cleaning effect.

These copper plated aluminum-based structures, with pores in the otherwise insulating oxide filled with conductive copper metal, have the potential to serve as electrical bars as junctions between pure-copper based bars and aluminum-based bars in high power applications, as for example in off-shore windmill farms.

No procedure is available to deposit a film of pure copper directly on an unanodized surface of aluminum. However, one report (Synthesis of Biomimetic Superhydrophobic Surface through Electrochemical Deposition on Porous Alumina, Jiadao Wang, Ang Li, Haosheng Chen, Darong Chen, Journal of Bionic Engineering, 8 (2011) 122-128) claims this is possible. Such copper films are however normally unstable on the aluminum surface and can be washed off the surface with a jet of water i.e. they are mechanical unstable from a practical point of view.

The present inventor has developed a more solid foundation than available earlier, for optimization of the pore filling of anodic aluminum oxide (called 'alumina') on substrates of pure aluminum, or alloys, with copper, using electrochemical methods.

With reference to the current status in the literature and patents, it seems that the invention is first in having obtained optimal control over pore filling of the porous oxide on a substrate of aluminum based on DC methods, a procedure which seems untenable from the existing literature.

It has also been found a method not falling within the scope of the claims, based on 50 Hz sinusoidal AC and with this method obtained a good way of achieving pore filling, including overgrowth of copper and subsequent removal of the overgrown film. Using 50 Hz sinusoidal activation was deemed less than optimal from the most credible sources in the literature. A particular issue is to avoid the adsorption of any metallic copper from the electrolyte on the upper surface or on edges and ridges during the process, which may create shorting paths for the ion current, resulting in less- and spread filling of the pores. This may be avoided by interrupting the deposition process and scrubbing of the surface to remove such deposits.

The inventor has also studied the growth of copper deposits on the surface beyond the pore filling and apparently for the first time discovered some important trends with respect to AC versus DC methods, and as functions of the voltages used for the deposition compared to the voltage used for the anodization. AC methods at all voltages tried may deposit copper films consisting of micro-clusters and cubic crystals that are unstable and loosely connected to the oxide surface. The process etches the surface of the oxide.

At high voltages (above 10 V here) DC grown films are composed of copper that flow out of the pores forming a continuous material without damages to the underlying oxide surface. This growth mode requires high ion currents, at the level of 1 A/cm² and may cause too much heating. Thus, intermittent processing might be needed, or one might envision to perform local processing of smaller parts of larger items by a suitably controlled and moveable electrode for industrial use of the process. The local deposition of copper may be performed by moveable electrodes within a larger container with an object to be coated with copper, the moveable electrodes may be moved or displaced by robotic means, such as robotics arms immersed in the electrochemical fluid within the container.

At lower voltages, a process was developed in which DC grown films get composed of uniformly sized micro-clusters of copper nanowires or lamellae with roots in the pores. Precisely this kind of film has obvious uses both as an antibacterial/viral coating on aluminum/alumina, after mechanical polishing to obtain a smooth surface. Alternatively, this may be applied for electrical connections between copper elements and aluminum based elements, for example in the off-shore industry.

### Definitions

In the context of the present invention, the term 'copper film' comprises primarily metallic copper, i.e. copper in valence zero, but the copper film may also comprises other copper compounds, Cu oxide, for example in the surface two atomic layer, and to some extent impurities, such as Al203.

In the context of the present invention, the term 'thin film' is understood by the skilled person so include films below a some or few micrometers, such as below 10 micrometer, or below 5 micrometers, such as below 1 micrometer, preferably in the range of 50-200 nm, preferably 75-150 nm.

In the context of the present invention, the term 'aluminum alloy' (British English spelling aluminium alloy) is to be understood as an alloy comprising primarily aluminum, Al, but other elements may be present to some extent, especially silicon (Si), oxygen (O), copper (Cu), magnesium (Mg), manganese (Mn), tin (Sn) and zinc (Zn). In the context of the present invention, the term 'aluminum alloy' may particularly include a pure, or substantially pure, aluminum substrate i.e. without any other element than Al.

In the context of the present invention, the term 'anodization' (British English spelling anodisation) is understood by the skilled person to include an electrolytic passivation process applied to increase the especially the thickness, but also one, or more, chemical or physical properties, and/or the surface structure (incl. coloring) of an oxide layer on the surface of the metal i.e. in this context of the aluminum, or the aluminum alloy. Traditionally, it is named so because the metal, e.g. aluminum, to be treated forms the anode electrode of an electrochemical cell, but the present invention is not limited to the convention, if the anodization process can be performed oppositely, or differently, in the sense that an oxide layer is grown. Thus, in the context of the present invention the anodization of the aluminum alloy substrate results in a regularly ordered porous aluminum oxide (PAO) film; this PAO film and the remaining aluminum alloy thereby forming a combined PAO/aluminum substrate, as the skilled person in electrochemistry, especially anodization, will immediately understand. In this patent application, the term 'substrate' may accordingly be applied both for the combined PAO/aluminum substrate, and for the aluminum, or aluminum alloy, substrate alone (i.e. without the PAO) depending on the context. In particular because the PAO film, or interlayer, is typically relatively thin compared with the aluminum substrate, i.e. there is typically several orders of magnitude difference between a several micrometer, or thinner, thick PAO film and the underlying aluminum alloy substrate, which normally may have macroscopic dimensions.

In the context of the present invention, the term 'stable film' is understood by the skilled person to include films that are electrochemically stable unless indicated otherwise or understood otherwise from the context, i.e. a stable film in this context will not galvanic corrode under such conditions or tests usually applied by the skilled person for this purpose.

In the context of the present invention, the term 'alternating current (AC)' is understood by the skilled person to include an electrochemical deposition with an alternating polarity over time, typically indicated by a frequency (Hz) or similar measure. It is to be understood that for relatively slowly varying AC, there may be gradual transition to an effective direct current (DC) situation. Similarly, in the context of the present invention, the term 'direct current (DC)' is understood by the skilled person to include also pulsed DC electrochemical deposition (PDC), with varying pulse width modulation (PWM) and duty-cycle, even though more than just the voltage is varied for PDC.

In the context of the present invention, the term 'adhesion' of a film on a substrate is understood by the skilled person to include films that will stay on the supporting substrate during normal procedures and tests for adhesion, e.g. films will adhere to the substrate after blasting, grinding, polishing, and other kinds of mechanical tests/treatments to measure adhesion, both quantitative (e.g. a maximum pressure) and qualitative (e.g. "good" vs bad" adhesion), etc.

In preferred embodiments, a further anodization is performed to create a regularly ordered PAO film on the aluminum alloy substrate. Thus, for example a two step anodization may be performed, in particularly so that at the end of the combined anodization process the pore in the top surface are further opened. Further steps of anodization are also contemplated, e.g. three, four, five, etc. steps of anodization may be performed. Thus, this may result in more opening of the pores, and a high number of open pores, which may result in better adhesion of the copper film.

In other preferred embodiments, the process for manufacturing a stable copper film on a PAO/aluminum substrate may have an anodization, which comprises a gradual lowering of the anodization voltage from a first voltage level (V1) to a second voltage level (V2), the second voltage level being lower than the first voltage level, and wherein the second voltage level is kept substantially constant for a predefined period of time (T_V2), wherein the first and the second voltage level, the predefined period of time (T_V2) and/or the ramping rate (dV/dt) between the first and the second voltage level is adjusted for controlling the thickness (d) of the PAO from the bottom of the pores down to the aluminum alloy substrate. This thickness is normally called a 'barrier oxide layer'. This oxide has a structure and composition different from the oxide in the pore walls, which also contains hydroxides and rests of the anodizing electrolyte. The technical effect is slightly disordered PAO growth with lowered thickness of oxide at bottom of pores, which may improve the mechanism for ionic attraction of copper ions into the pores, and in turn creating a better adhesion of the thin copper film to the substrate through connection with the copper deposited in the pores. This is in contrast to copper settling on top of the PAO. The invention is particular in that the barrier oxide layer, in particular the thickness d, has been optimized for improved adhesion and electrochemical stability of the copper film on the substrate.

For producing a copper film on a PAO/aluminum substrate, electrodepositing copper on said substrate so as to deposit metallic copper 1) in the open pores of the substrate may be performed at a pH below or around 3, as it will be explained below in more details. It may, however, also be advantageous to apply the invention at other pH intervals, e.g. below or around 5, alternatively preferably at a pH below or around 4.

Advantageously, the process for manufacturing a copper film on a PAO/aluminum substrate with electrodepositing copper on said substrate so as to deposit metallic copper 1) in the open pores of the substrate may be performed with sufficient stirring and/or sufficient high and/or properly chosen DC voltage to reduce gas evolution in the pores. More particularly, it may be performed so that copper is electrodeposited primarily on the inner surface of the pores before a central volume of the pores is filled with copper. It is observed that reduced gas evolution takes place when using DC currents. Thus, with DC applied, it is contemplated that H2 gas will evolve at the bottom of the pores, whereas in the prior art, the use of AC may result in both H2 and O2 gas evolution, which may have a negative influence on the copper growth down in, or within, the pores.

In some variants not covered by the claims, the process for manufacturing a copper film on a PAO/aluminum substrate for electrodepositing copper on said substrate may comprise the use of alternating current (AC) - instead of direct current (DC) - for depositing 1) in the open pores of the substrate.

In other variants, in particular suited for industrial upscaling of the invention, the substrate - between and/or during any of the steps for electrodepositing copper 1) in the open pores of the substrate, and 2) on the top of the substrate so as to form a copper film on top of the substrate - may be mechanically treated to remove, at least partly, any copper electrodeposited on top of the substrate before the pores are filled with copper, for example by polishing, grinding, blasting, brushing, or other kind of abrasive treatments such as violent stirring during intermissions of the deposition procedures, etc. for better growth, in particular adhesion, of the final resulting copper film.

The process for manufacturing a copper film on a PAO/aluminum substrate for electrodepositing copper on said substrate 2) to form a copper film on top of the substrate may result in an average film thickness in the interval of 50-200 nm, preferably 75-150 nm, though other thicknesses are also contemplated. If the pores are filled with copper and limited overgrowth on top of the pores takes place, the thickness may in this sub-micrometer range, e.g. 50-200 nm thick copper. The advantage of these thickness intervals are reduced visual effect (e.g. difficult to see the copper layer with a normal human eye), while maintaining the functionality of the copper on the surface, i.e. antibacterial/viral effect. It may be mentioned that if mechanical post-treatment, e.g. polishing, is required, the required thickness of the thin copper film will usually be in the range of at least some micrometers (µm), e.g. at least 2 micrometers, at least 4 micrometers, or at least 6 micrometers, to last during the mechanical post-treatment after the electrodeposition.

It is also possible to make thicker layers of copper on a PAO/aluminum substrate for electrodepositing copper on said substrate so that forming a copper film on top of the substrate may result in an average film thickness in the interval of 50-200 micrometers as grown, though lower thicknesses are resulting after mechanical polishing. If the pores are filled with copper and limited overgrowth on top of the pores takes place, the thickness may be in a lower-micrometer range, e.g. 20-50 micrometer thick copper. It may be mentioned that if mechanical post-treatment, e.g. polishing, is required, the required thickness of the thin copper film will usually be in the range of at least some hundred micrometers (µm), e.g. typically 200 micrometers, or at least 400 micrometers, to last during the mechanical post-treatment after the electrodeposition.

The structure of the copper film may vary significantly with processing voltage. Stable films, adhering well to the surface, through connection with the copper filled pores, are grown using DC methods. Microcrystalline films with well-connected, similarly sized, spherical crystals may be formed at lower voltages, versus stable uniformly structured compact copper films formed as the other extreme, at higher voltages, as the formation process has been found being strongly dependent on the growth voltage conditions.

Advantageously, the process for manufacturing a copper film on a PAO/aluminum substrate with wherein electrodepositing copper on said substrate may be performed around 10-15 V, or higher, so as to deposit metallic copper 2) on the top of the substrate so as to form a substantially continuous copper film connected to the copper in the pores of the substrate. In this context, the film may be completely continuous i.e. the coverage of the film on the underlying may be 100%, or close to 100%, but in this context it will also be understood that substantially continuous may imply an (area) coverage of at least 70%, 80%, or 90%. From a functional point of view, the continuous film is related to the adhesion of the film, the better coverage normally resulting in improved adhesion of the copper film.

In one embodiment, the process for manufacturing a copper film on a PAO/aluminum substrate for electrodepositing copper on said substrate may be performed below 10 V and with a current density below 0.05 A/cm2, so as to deposit metallic copper 2) on the top of the substrate so as to form a substantially continuous copper film, which will be explained in more detail below.

In another embodiment, the process for manufacturing a copper film on a PAO/aluminum substrate for electrodepositing copper on said substrate may be performed above 10 V with a current density above 0.15 A/cm2, so as to deposit metallic copper 2) on the top of the substrate so as to form a substantially continuous copper film, which will be explained in more detail below.

In another embodiment, the process for manufacturing a copper film on a PAO/aluminum substrate for electrodepositing copper on said substrate may performed below 10 V and with a current density above 0.05 A/cm2, so as to deposit metallic copper 2) on the top of the substrate so as to grow micro-cluster copper, the micro-clusters having groups of copper nano-wires and/or copper lamellae. These micro-clusters may be liberated from the pores through an etching effect, etching away the top layer of the PAO below the clusters, or by other mechanical removal procedures.

In yet another embodiment, the process for manufacturing a copper film on a PAO/aluminum substrate for electrodepositing copper on said substrate may performed below 10 V and with a current density below 0.15 A/cm2, so as to deposit metallic copper 2) on the top of the substrate growing out of the pores as micro-cluster copper, the micro-clusters having groups of copper nano-wires and/or copper lamellae.

In a second aspect not covered by the claims, the present disclosure relates to an object comprising a PAO/aluminum substrate with a copper coating manufactured by the process of the first aspect. Such objects may include, but is not limited;
- part of a building, such as a doorknob, a handle, a railing, and other building equipment where human skin, e.g. the hand, touches the surface during normal use, or water taps, and other kind of bath room equipment where the human skin, e.g. the hand, touches the surface during normal use, etc.
- parts or pieces for health equipment, or medical devices, in particular equipment /device, where human skin is intended for touching and/or risk touching the surface during normal use, and/or
- a current conductor, preferably for high-power applications, such as a bus-bar, for example in renewable energy applications; like solar energy, hydro energy, wind energy, etc.

In a third aspect, the invention related to the use of the process for manufacturing a copper film on a PAO substrate according to the first aspect for growing nano-crystalline copper alloys on the PAO surface. Such nano-crystalline copper structures may have catalytic purposes, related to COz and hydrogen reactions, for example, due to the high-surface-to volume-ratio observed in these structures.

The first, second and third aspect of the present invention may each be combined with any of the other aspects. These and other aspects of the invention will be apparent from and elucidated with reference to the embodiments described hereinafter.

### BRIEF DESCRIPTION OF THE FIGURES

The invention will now be described in more detail with regard to the accompanying figures. The figures show one way of implementing the present invention and is not to be construed as being limiting to other possible embodiments falling within the scope of the attached claim set.
Figure 1 is a schematic drawing of an object manufacturing according to the invention,
Figures 2 and 3 are schematic drawings of the porous aluminum oxide (PAO) according to the invention,
Figure 4 is a schematic drawing of the copper film growth on the porous aluminum oxide (PAO) according to the invention,
Figure 5 is a schematic drawing of the resulting copper film on the porous aluminum oxide (PAO) according to the invention,
Figure 6 is schematic graph of the voltage as a function of time during anodization according to the present invention,
Figures 7-23 shows various microscopic views using SEM and AFM related to the invention, and
Figure 24 is a flow-chart of a method according to the invention.
Figure 25 shows a graph illustrating the measured relationship between the voltage and the current density at the beginning of the deposition process.
Figure 26 shows an image of an aluminum substrate where the copper has been removed.

### DETAILED DESCRIPTION OF AN EMBODIMENT

For the most efficient processing results with copper ions, the pore structure of the oxide after anodization must be as well ordered as possible with a uniform depth of the pores across the treated surface to arrive at a uniform filling of the pores. As demonstrated in "Multigram synthesis of copper nanowires using ac electrodeposition into porous aluminum oxide", Genaro A. Gelves, Zakari T. M. Murakami, Matthew J. Krantz and Joel Haber, Journal of Materials Chemistry, 2006, 16, 3075-3083, it is very difficult to obtain a reasonably uniform level of pore filling across a larger area. Therefore, to improve on this situation was the primary aim of the present investigations, resulting in a successful outcome, as demonstrated here.

The invention applies a two-step anodization process both with pure-aluminum sheets and for alloy items. This process, as described in detail below and with suitable pre-processing steps, gives the most well-ordered and regular pore structure with uniform depths across larger surface areas, which may be a necessary precondition for a success when aiming at a uniform filling. All of the currently available literature, about the filling of pores in the oxide created on a substrate of aluminum with metals, is mainly advocating the use of AC- or tailored AC methods. However, Gelves et al. found that pore filling was only moderately efficient when applying 200 Hz sinusoidal AC power to a larger set of aluminum sheets to produce gram quantities of copper nanowires in the pores. To them, this represented the optimal processing parameters. The length of the liberated nanowires thus varied considerably, if the porous oxide surface with the filled pores had not first been levelled down a large amount, compared to the pore depths, by ion etching methods, thus emphasizing the difficulty of arriving at a uniform filling to the top of the pores. They otherwise followed the same procedures with two step anodization but used sulphuric acid instead of oxalic acid and used pure aluminum sheets for their experiments. Sulphuric acid is most commonly used in the anodizing industry. It typically results in narrower pores than oxalic acid during anodization. However, the inventor does not assume any major differences between these media, but stick to oxalic acid in the present investigations, as it allows a better control of the processes with the microscopic tools used here, i.e. a scanning electron microscope (SEM) with X-ray detector for energy dispersive elemental X-ray spectroscopy (EDS) and an atomic force microscope (AFM), which is used in non-contact tapping mode.

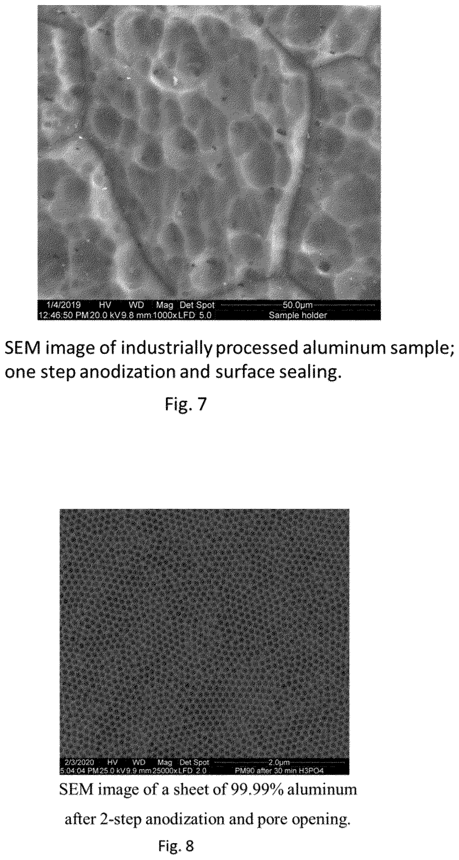

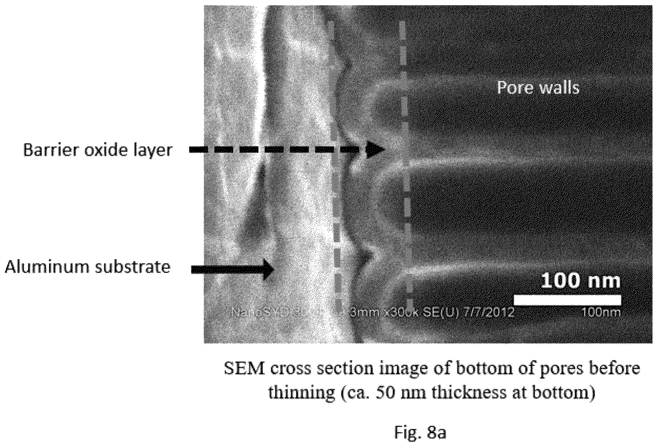

As in many other reports, it was discussed by Gelves et al., how the electric field strength inside the pores is critical to effectively attract metal ions to the bottom of the pores. In the present case the strength of the field forming the anodized pore structure, was set up by applying a constant voltage of 40 V between the aluminum sheet (as the positive terminal) and the negative electrode in the electrolyte. This gives a steady "growth" rate and anodizing current after a short, strong peak in the current due to the formation of the barrier oxide layer on top of the pristine aluminum surface at the bottom of the pores. This barrier oxide layer propagates inwards into the aluminum, staying as the bottom oxide during the formation and deepening of the pores. In other studies, the current is held constant. A field strength corresponding to this 40 V voltage in the setup had to be expected to be necessary for attracting copper ions from the solution to the bottom of the pores. This would be a relatively strong field and would create a high current with a corresponding high heat dissipation during the process, which might damage the porous oxide structure. Thus, the method adopted in the present case, to avoid these inconveniences, is to thin the bottom barrier oxide layer at the end the anodization process by a gradual lowering of the voltage during the final step of the anodization. In this case one ends at a value of 10 V, which is kept for some time to assure a steady state during the finishing of the growth. This causes the oxide at the bottom of the pores - the so-called barrier oxide - to become slightly disordered, but thinner than the oxide, which would normally result by keeping the anodization voltage at 40 V until the end of the process. See figure 8a.

As a final step the pore openings may be widened by immersing the processed items in 0.3 M phosphoric acid. The degree of opening obtained depends on the processing time.

By immersing the processed item in phosphoric acid for 15 to 60 minutes, and increase temperature as needed, the pore diameter may typically be increased from 75 nm to 90 nm at the top surface.

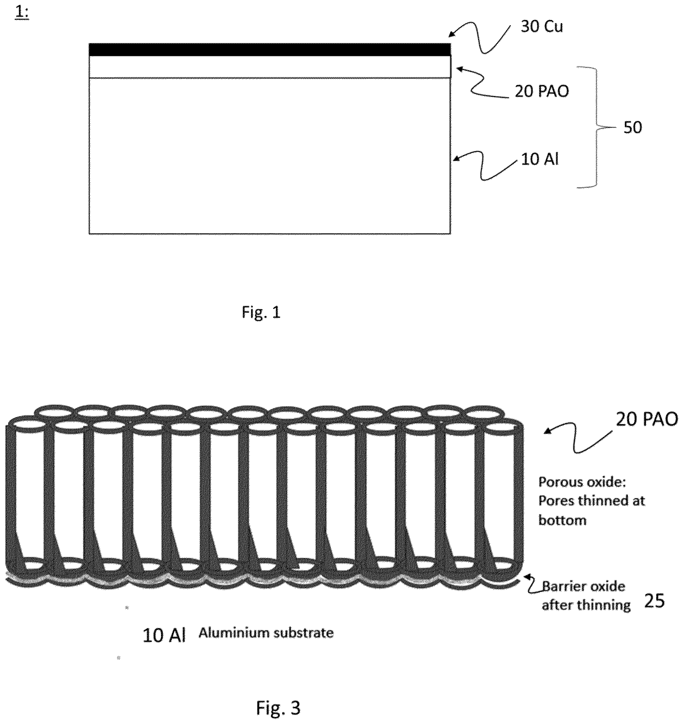

Figure 1 is a schematic drawing of an object 1 manufacturing according to the invention with a process for manufacturing a thin and stable copper 30 Cu on a porous aluminum oxide film PAO 20 on an aluminum alloy substrate 10. The process comprising anodization of the aluminum alloy substrate resulting in a regularly ordered PAO film; the PAO film and the remaining aluminum alloy thereby forming a combined or resulting PAO/aluminum substrate 50. This is generally known from the field of anodization within electrochemistry.

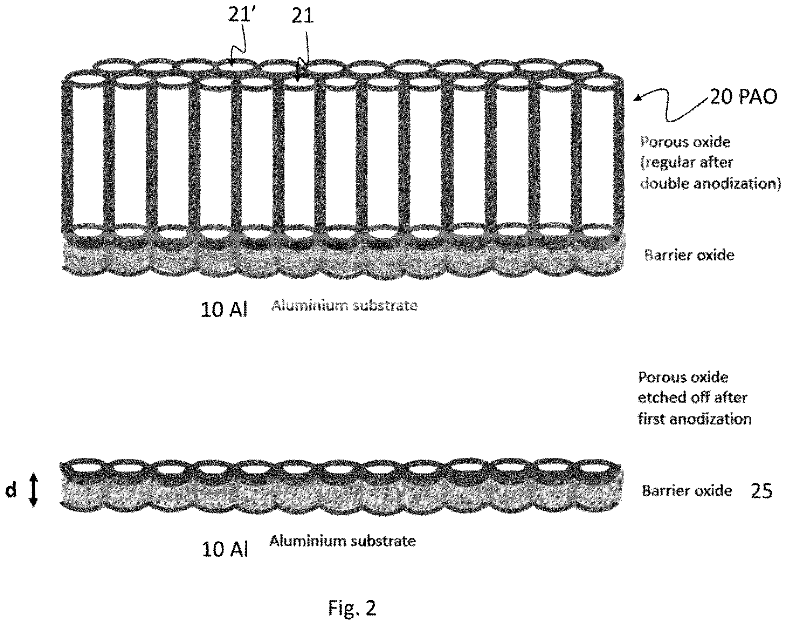

Figures 2 and 3 are schematic drawings of the porous aluminum oxide (PAO) according to the invention during the various steps of multiple anodization and optional etching.

Thus, in Figure 2, upper part, a PAO 20 structure is schematically seen after a double anodization with individual pores 21 and 21', whereas in the lower part, the PAO 20 structure is schematically seen to have lower height due to an etching performed. Also schematically seen is the barrier oxide 25 i.e. the layer between the bottom of the pores and the upper part of the aluminum substrate 10.

The etching of said PAO/aluminum substrate is performed for further opening of the top of the pores in the surface of said PAO film after the one or more anodization steps. Details of this etching will be explained below. Schematically indicated in the lower part of Figure 2, the thickness of the barrier oxide layer d is shown, which may be important for both the adhesion of the copper film and electrochemical stability.

In Figure 3, it is schematically indicated how the pores at the bottom are thinned at the bottom of the pores, the thinning being performed using a sufficiently low voltage, e.g. 10 V. The thinning is commonly understood to be active due to irregularly pore formation at the bottom of the oxides, the effect was first described and published by the research group lead by Gösele.

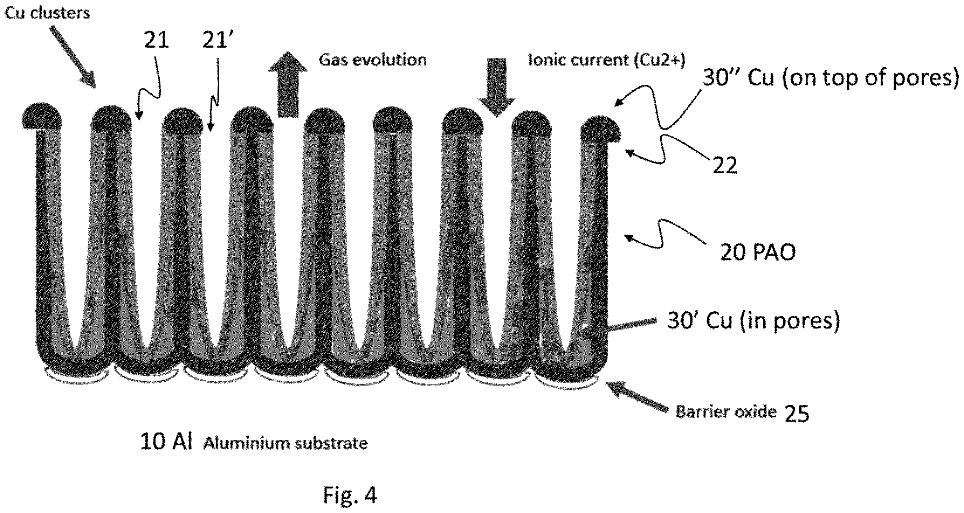

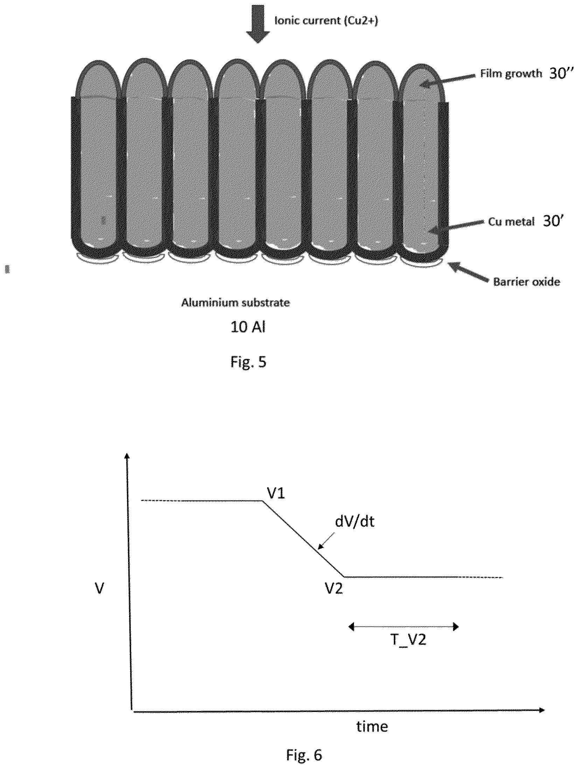

Figure 4 is a schematic drawing of the copper film growth on the porous aluminum oxide (PAO) according to the invention, where electrodepositing copper on the combined PAO/aluminum substrate 50, the electrodepositing comprises the use of direct current DC, preferably in the interval of 5-20 V, more preferably 10-15 V, so as to deposit metallic copper, Cu. The deposition of copper 30 and 30' takes places in both
1) in the open pores, generally 20, specifically 21 and 21', of the regularly ordered PAO film - at the same time or later - and
2) on the top of the regularly ordered PAO film, or layer, cf. arrow 22, so as to form a copper film 30 on top of the PAO film, cf. also Figure 5. The core of the invention is that the anodization is performed so as to improve the both adhesion, i.e. will stay on after e.g. polishing, and electrochemical corrosion stability, i.e. little or no galvanic corrosion will occur, of the electrodeposited copper on said PAO/aluminum substrate.

Notice that some copper 30" may be deposited on top of the pores 22 while growing copper 30' in the pores, too, as schematically in Figure 4. In some embodiments, this top copper may be removed mechanically, e.g. by grinding or polishing or it may be removed during intermissions of the deposition process by brushing or violent stirring.

In some embodiments, electrodepositing copper on the substrate so as to deposit metallic copper 1) in the open pores of the substrate is performed with sufficient stirring and/or properly chosen and/or sufficient high DC voltage to reduce gas evolution in the pores as indicated in figure 4 by the solid outgoing arrow.

It is contemplated that the copper is electrodeposited primarily on the inner surface of the pores before a central volume of the pores is filled with copper.

To reduce gas evolution the use of DC currents is preferred. Thus, when using DC voltages, it seems that hydrogen gas is primarily formed on the bottom of the pores.

Figure 5 is a schematic drawing of the resulting electrodeposited copper film 30 on the porous aluminum oxide (PAO) according to the invention. The copper film grows from the pores as also shown in Figure 4, and when the pores are - to a large extent - filled with copper, the copper grows from the pores to form a continuous, at least substantially continuous, copper film 32 as indicated in Figure 5.

Figure 6 is schematic graph of the voltage as a function of time during anodization according to the present invention. Thus, the anodization comprises a final, gradual lowering of the anodization voltage from a first voltage level V1 to a second voltage level V2, the second voltage level being lower than the first voltage level, and wherein the second voltage level is kept substantially constant for a predefined period of time, T_V2, wherein the first and the second voltage level, the predefined period of time T_V2 and/or the so-called ramping rate (dV/dt) between the first and the second voltage level is adjusted for controlling the thickness of the PAO from the bottom of the pores down to the aluminum alloy substrate, i.e. the so-called barrier oxide layer. (See figure 8a.) This oxide has a structure and composition different from the oxide in the pore walls, which also contains hydroxides and rests of the anodizing electrolyte. The effect is a slightly disordered PAO growth with lowered thickness of oxide at bottom of pores. This may improve the mechanism for ionic attraction of copper ions into the pores, and creating a better adhesion of the thin copper film to the substrate through connection with the copper deposited in the pores, in contrast to copper settling on top of the PAO.

### Experimental procedures:

### The anodization process may have the following steps:

The virgin aluminum sample is first cleaned and degreased by washing with soap (and for smaller samples) in acetone. If the item is rough, a mechanical surface polishing may be required. Pure aluminum sheets are annealed at 500°C in a furnace with air for a few hours. Larger alloyed pieces are not annealed, as they have had a certain thermal history to obtain certain properties of the bulk material.

One then employs electropolishing to produce a smooth and shiny surface, using a suitable electrolyte and voltage/current programs. Before the items are electropolished they are dipped shortly (about 1 min.) in 3 M NaOH, washed in water (1 min.) and then neutralized in 5.5 M HNO3 (1 min). They are then washed in water and quickly entered in the electropolishing system. A good electropolishing result for small areas (2 cm²) is obtained using a Struers Lectropol-5 equipment with Struers electropolishing liquid for aluminum, at 24 V for 30 sec and at 250°C. For larger items, with several surfaces the inventor used a double walled, cooled glass container with the same electrolyte, and a voltage of 24 V at a current limit set at 10 A. Typical polishing times were 30 sec, or until a shiny surface is obtained.

After electropolishing, anodization was carried out in a double walled cooled glass container, held at 0°C, with a relatively vigorous continuous magnetic stirring. The anodization was done under constant voltage conditions, using 0.3 M oxalic acid at a voltage of 40 V ("mild anodization"). This creates a pore structure with pores of around 100 nm center-to-center distance or in diameter. The anodization process starts at defects and impurities at the surface, but as the anodization proceeds to etch into the aluminum, the pore growth stabilizes in a self-organizing mode with a regular pattern structure and expansion of the oxide beyond the original surface, because of its atomic structure and because of re-deposition of some of the dissolved aluminum bound to oxygen and acid residues in the pore walls.

In industrial processing only this step with one anodization cycle is normally used. After obtaining the desired thickness, after a calculated process time, this oxide looks disordered in the top surface layers, but it is well ordered further down into the structure. The processed item is then treated in different ways in the normal practice either to fill the pores with colorants or just to close the pores, in which case the ordering is not important. For alloys the anodization often causes a pattern of cracks to form in the surface because the oxide expands beyond the strength of the aluminum substrate. After sealing the pores these cracks are still present and cause the protection of the surface to be less perfect than if these cracks and ridges had not been created. An example of an industrially processed surface is given in Figure 7.

In the present case, looking for the best way to structure the pore pattern, the inventor removed the upper, less well-ordered part of the anodized oxide structure from the initial step by etching the oxide in a stirred mixture of chromic acid and phosphoric acid at 70°C (often called PC etch).

This etching step may be continued for times long enough to completely remove the porous oxide, leaving an imprint of the best ordered pore structure near the aluminum substrate, covered by a differently structured and denser oxide, called the barrier oxide. This oxide is not attacked by the PC etch. This complete etching of the porous oxide is not always necessary, but sufficient etching time must be used to etch away the least ordered part of the porous oxide. The next step is to repeat the anodization, to obtain a desired pore depth / thickness of the porous oxide layer.

The actual growth rates for different processing parameters can be evaluated by looking at cross sections of pores on the substrate with an electron microscope or by using ultrasound-based tools used to measure the thickness of the oxide layers (less precise, but often used in the industrial processing for quality control). Obviously, the depth of the pores is an important parameter for adjusting the filling process. Reliable data for different growth conditions is also available in the literature.

At the end of the second anodization step, the anodization voltage is ramped down from 40 V to 10 V over a period of 30 min, and then kept at 10 V for 30 min. With this voltage in mind, it is easy to assume that the necessary voltage to create a sufficient driving electric field at the bottom of the pores would be at least 10 V.

The total duration of the 3 steps including the ramping may be about 24 hours. After each step the object may be washed in an ultrasonic bath with de-ionized water in about five minutes. That is after the first anodization, the etching and the second anodization including the ramping. Then the object may be stored in alcohol until the next step to keep the pores clean.

If pore opening is necessary, this will now be performed, as mentioned earlier. The item is now washed in water and dried before the next step, the filling of the pores. A finished sample, after pore opening, as imaged with the electron microscope is seen in Fig. 8. To image this sample, with an insulating nature, a neutralizing background pressure of water gas was used inside the sample compartment of the electron microscope.

A pore debt of 25 micrometer, is a good starting point for a copper filling with a long service life, as well as resulting in a mechanically and chemically strong surface coating of the porous oxide layer.

Fig. 8a shows a SEM cross section image of bottoms of pores before thinning. There shows a barrier oxide layer between the pores and the aluminum substrate.

### Filling of the pores with copper metal:

A bath of 0.5 M CuSO4 and 0.258 M boric acid is used as the copper ion donor solution with a pure copper or stainless-steel counter electrode without cooling of the bath. The counter electrodes have been shaped to cover the round glass vessel inner surface to allow for differently shaped items to be processed on all sides in the center of the vessel. It is assumed that shape and distance of this electrode from the sample is not critical because the active field region should be localized in the electrolytic double layer above the aluminum with the thinnest oxide layer, which is at the bottom of the pores. This is most clearly seen with alloy samples with cracks formed in the oxide. On these samples copper clearly starts to be deposited inside these cracks.

The temperature of the bath should be around room temperature (20 °C) as heating can course changes in the surface structure or the porous aluminum oxide.

Important contributions to this field by Gosele et al. (Nielsch, K., Müller, F., Li, A.-P. and Gosele, U. (2000) Uniform Nickel Deposition into Ordered Alumina Pores by Pulsed Electrodeposition. Advanced Materials, 12, 582-586. http://dx.doi.org/10.1002/(SICI) 1521-4095(200004) 12: 8< 582: :AID-ADMA582>3.0.CO;2-3) have concluded that the most efficient method for filling the pores on top of the aluminum substrate with metal from ions would be based on AC- (alternating current) or pulsed variable polarity voltages, and the thinning of the oxide at the bottom of the pores. In another branch of this field aiming at creating metallic nanowires, the porous oxide is initially stripped from the aluminum substrate as a self-standing entity, by dissolution of the aluminum. This free-standing pore structure or membrane is then used for electrolytic filling of the pores using DC (direct current) methods with relatively low voltages and currents, by depositing a conducting and continuous gold film on one side of the porous membrane and using this gold layer as the electrode at the bottom of the pores without the problem of an insulating barrier oxide layer. This method has been found to work well and to be able to fill the pores relatively uniformly.

However, the inventor has succeeded to make use of the DC method also for filling the pores grown on an aluminum substrate and compared it with the AC method. One argument in favor of both methods without preferences is actually due to the so-called valve-metal characteristic of aluminum. This means that during the change of direction of the field inside the pores using AC methods, there is an asymmetry in the direction of the ionic current flow, or rectification effect, such that there is a higher net current of positive ions flowing towards the bottom of the pores when the aluminum is biased negatively with respect to the other electrode (and the electrolyte). This would then look like an effective DC current situation. Considering the electrolyte content of water, the changing polarity in AC would also cause water molecules to split at voltages above 1.25 V, which would release oxygen and hydrogen, respectively, inside the pores, at the two opposite polarities. With a DC voltage, only hydrogen would be released inside the pores while oxygen is released at the other electrode.

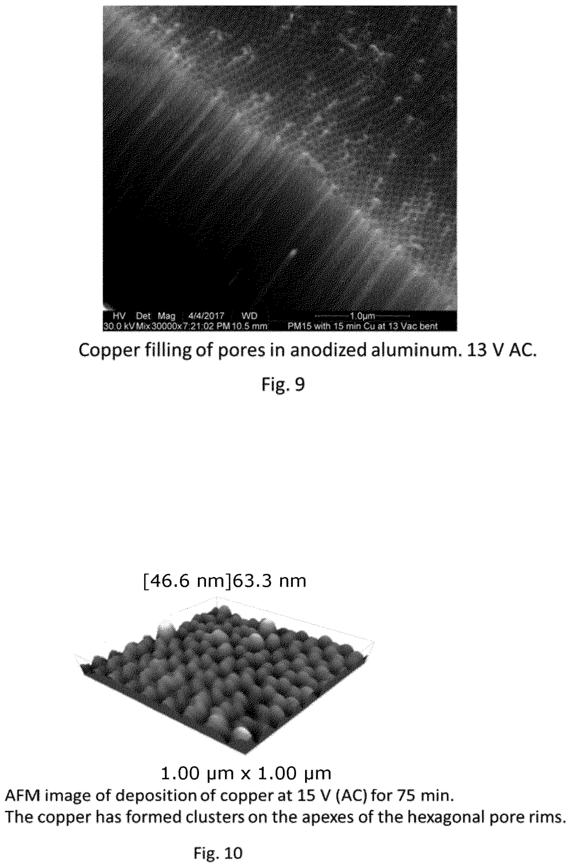

To obtain filling of the pores it is to be expected that voltages equal to or above 10 V DC or 10 V(rms) AC would be required. Fig. 9 shows an example of an attempt at filling of the pores using 13 V(rms) AC for 15 min., not according to the invention. However, the process was also found to work at lower voltages, but with variable results, depending on the voltage.

The image was recorded with an electron microscope, without any addition of conductive material or neutralizing gas to allow imaging without charging of the sample. The brighter material in the images is copper, which has filled the pores. The filling is not complete, as seen from above the surface, but the surface is rendered conductive due to the presence of copper. Without copper in the pores imaging without neutralizing the charge building up on the insulating surface would not be possible.

A more complete filling level is obtained by continuing the deposition, and the inventor then searched for a method to identify the completion of the total filling by looking at changes in the voltage/current characteristics during deposition. This turned out not to be a practicable method because parasitic currents occurred from a disturbing effect in this method, the pile-up of copper on top of the surface, deposited randomly from small copper crystals floating around near the surface, and attaching to the surface, or from deposits of copper covering the rims of the pores. These two effects have been studied separately to try to eliminate them as impediments to the filling of the pores, by offering an electric path with a much higher affinity for copper deposits on top of the surface than inside the pores. Other pathways for the current are due to sharp edges and ridges into the surface. All these effects menace the growth of copper metal in the pores, as they offer lower resistance paths for the ions.

Another critical factor for the pore filling is the gas evolution from the splitting of the water molecules inside the pores, which is clearly seen by formation of gas bubbles on the surface. The effect of this can be minimized by vigorous stirring of the electrolyte, but from many images taken during the process it was concluded that the pores due to the gas evolution (at least in the case of 100 nm diameter pores obtained with oxalic acid) get covered primarily by copper on the inner pore surfaces, before the central volume gets occupied. This copper coverage grows out of the pores and forms clusters on the apexes of the pore rims. This is shown in the AFM image (Fig. 10) recorded of such a case. This image shows a 3D rendering of the recording obtained with an atomic force microscope. The height scale is exaggerated compared to the scales of the edges. The copper was deposited at 15 V 50 Hz AC for 75 min.

Similar results were obtained using DC deposition. The key to obtaining the most uniform filling of pores across a larger area is to keep copper from depositing outside the pores, and to keep the gas evolution as low as possible, which is controlled by keeping the pH of the solution around a value of 3, though other values around 2, 4, and 5 are contemplated. Good results have been obtained by using vigorous agitation, but the best and consistent results are obtained by intermittent deposition, where breaks in the process are used to remove "loose" copper from the outer surface, edges and rims, with cotton swaps. For industrial scale applications some means of keeping copper from depositing outside of the pores may be implemented by intermittent processing and scrubbing of the surface. In this case the ion current is stable and much lower than when current pathways are existing, which also reduces heating.

The pore filling can at this stage of development of the process best be ascertained by visual inspection during intermittent deposition cycles. The copper particles forming and scrubbed into the liquid during the process may be collected and may be used as catalysts. They assume different structures, as will be discussed below, and in some cases have large area to mass ratios, which should make them ideally suited for catalytic applications.

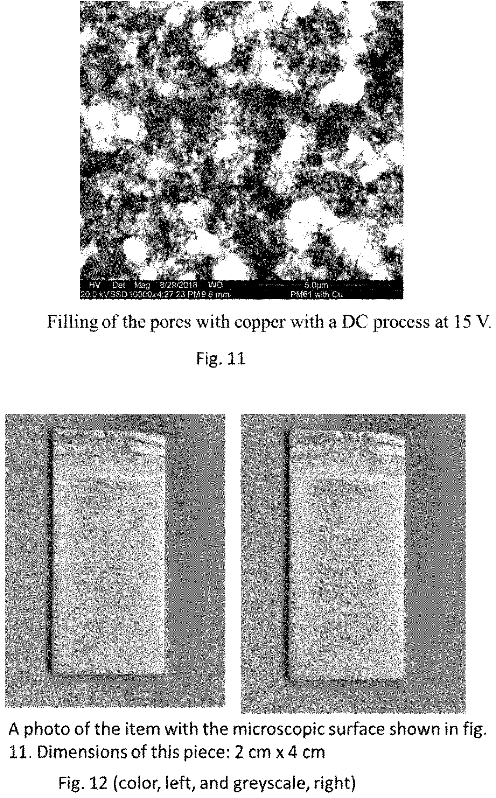

It was decided to try to use DC processes, as this would probably be easier to scale up to industrial applications, which will need relatively high currents for larger area items. For the pore filling one obtained almost similar performance if one observed the experience gained from the AC processing, requiring intermittent processing with removal of loosely adsorbed copper. An example of successful DC processing to fill the pores is shown in Fig. 11. In this case the pores have been filled and the process has been continued beyond this point, which has deposited copper on top of the pores. Copper appears white in this image, so it is clear that the pores are filled uniformly across the surface below the overlying copper "clouds". Even with the extra copper on this surface the color of the surface is greyish like for normally anodized aluminum, with only a very week tint of red-brown coloring seen in reflection.

It has previously been believed that such overgrowth could be an advantage, because a larger proportion of the surface would then most likely have had the pores been filled. The copper film was afterwards removed by dipping the sample in 0.6 M H3PO4 at 60°C for a short time.

It was found, however, that this procedure etches away some of the top of the oxide, and tends to seal the surface, so that the amount of copper exposed is thereby significantly reduced. The surface is also roughened. The procedure was found to work for AC deposited coverages, but not for DC deposition. The reasons for this have been investigated and is due to different structures of the growing copper coverages, as documented later.

A photographic image of this surface is shown in Fig. 12. The starting formation of a coverage of copper on top of the pores is observed at the microscopic level (Fig. 11).

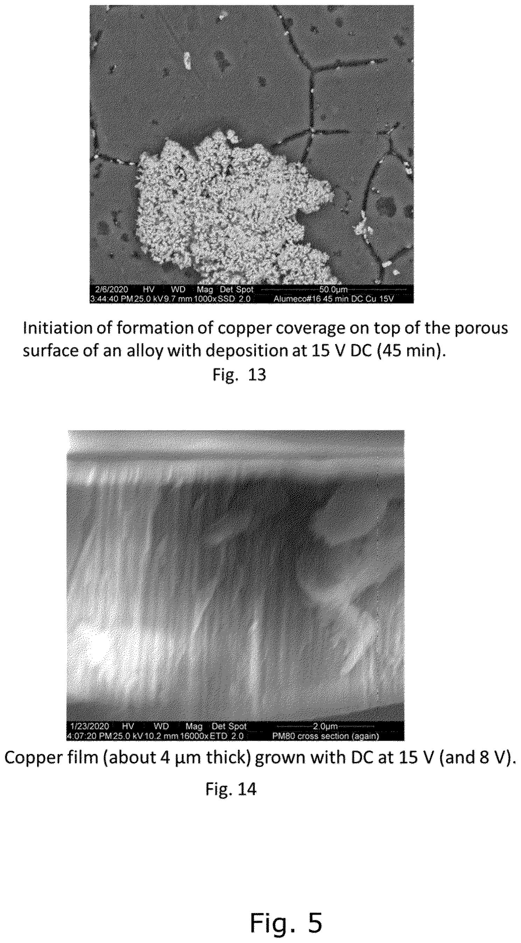

In Fig. 13 is shown how the initiation of the growth of a copper film with DC processing on an anodized alloy surface occurs at oxide pits and ridges formed during the anodization. Pores are not visible at this magnification but at higher magnifications they are seen as not uniformly filled due to local variations in the oxide thickness and the presence of sites favorable for the agglomeration of copper particles on top of the pores, such as in the ridges. Once such particles start to form they will act as nucleation centers with a higher local field attracting the copper ions.

### Growth and structure of the copper coverages on top of the surfaces, effect of the voltages; AC and DC methods

### High voltage DC:

The argument for using deposition voltages higher than the 10 V used for the oxide thinning phase of the growth of the porous oxide structure is corroborated by the experiments to obtain the best degree of pore filling. However, the processing at lower voltages was also found to deposit copper on the surfaces.

The corresponding lower currents could be an advantage for large scale processing, so a series of experiments was performed to understand the differences in the copper deposition mechanisms at various voltages below the critical limit (10 V) and above this value. There is an earlier report about the deposition of nickel and copper in the pores of anodized aluminum by Wang et al. where they used very low voltage DC (0.5 V) for 40 sec, based on anodization in phosphoric acid, and removal of the barrier oxide layer before the electrochemical deposition. Thus, the situation is closely similar to other methods with self-standing membranes covered with metal at the bottom of the pores to allow the deposition without a barrier layer of oxide. Under these anodization conditions the pores are of a much larger diameter and are less regular than in our case, as the anodization is only done with one step. Interestingly, and in agreement with our results mentioned later, they found the process leads to formation of micro-clusters of nanowires on top of the surface. They also found these structures formed on bare aluminum without pores, which is in direct contradiction to all other studies, including ours, as these copper wires can only be formed in the presence of pores, and as copper deposited directly on aluminum does not stick to the surface. Their conclusions were that the surfaces with the clusters of copper were super-hydrophobic. The present inventor will challenge this conclusion, or the arguments for it, claiming that the hydrophobicity would instead be due to the filling of the pores with metal. This situation would indeed give rise to so-called Cassie-state hydrophobicity, as claimed in the report by Wang et al.

### First a discussion of the growth of copper films on top of the pores with continued deposition past the filling of the pores, with DC processes:

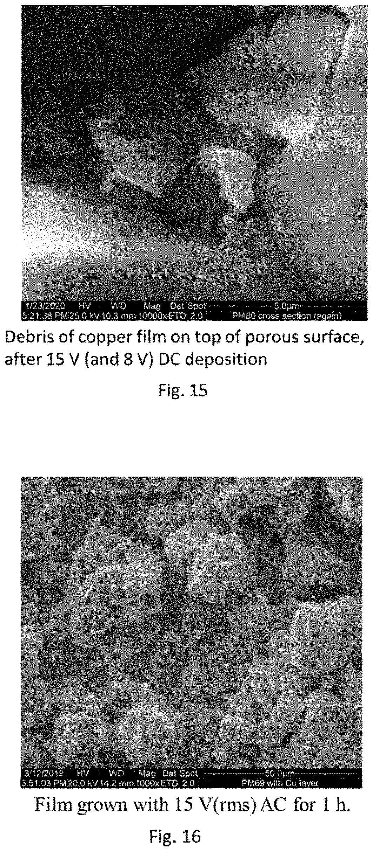

A copper film is shown in cross section in fig. 14, which was grown at a DC voltage of 15 V on a sample with pores opened (90 min in 6.5 M H3PO4) for 5 min at a current of 4 A/4 cm², and then at 8 V (2A/4 cm²) for 15 min. This produced a continuous film with an internal structure inherited from the pores. The transition to a lower voltage at the end of this process, and probably after filling the pores, was done primarily to reduce the heating of the electrochemical system. For further analysis, a small part of this film was then scratched off the surface to reveal the connection with the pores, as seen in the next image (fig. 15).

In this image it can be seen, how the copper has grown in and out of the pores, and how the film is structured by the pores. All the samples studied have pore depths around 40 micrometer (µm). Thus, the filling of pores at that depth is in itself a challenge, which the inventor has mastered with the present procedures, as demonstrated with this sample (figs. 14 and 15).

### High voltage AC (not covered by the claims):

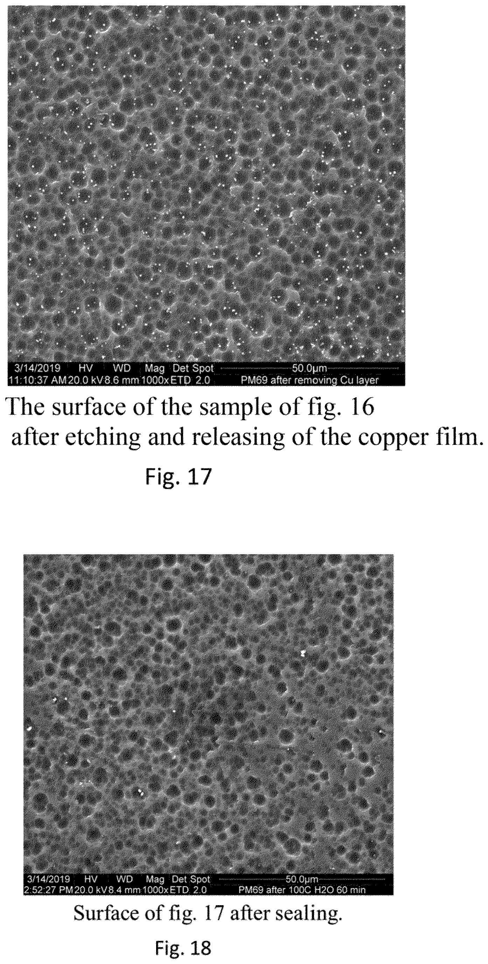

Film growth was also obtained at 15 V AC. Fig. 16 shows the structure of a film formed after 60 min deposition of copper at 15 V(rms) AC. The film consists of a conglomerate of clusters and crystallites. The clusters contain copper wires and the crystallites have the typical cubic structure of copper crystals, and they are not (blue) crystals of copper sulfate. The film has a very irregular structure and size distribution of the clusters and crystals. Following the proposal by Gelves et al., this film was removed from the surface by dipping the sample in hot phosphoric acid (50°C). This process works well, as described by them. The surface after removal of the copper film looked as seen in the next image, fig. 17.

The procedure etches into the surface to liberate the film and leaves a corrugated surface but still with visible copper fillings. As a last step, a procedure to seal the pores by dipping the sample in boiling water for 1 h (fig. 18) was used. The surface shown in fig. 18 still shows the presence of copper in the pores and may be used as and antimicrobial functionalized surface. It looks like a normally anodized surface without any coloring from the presence of copper.

### Low voltage DC:

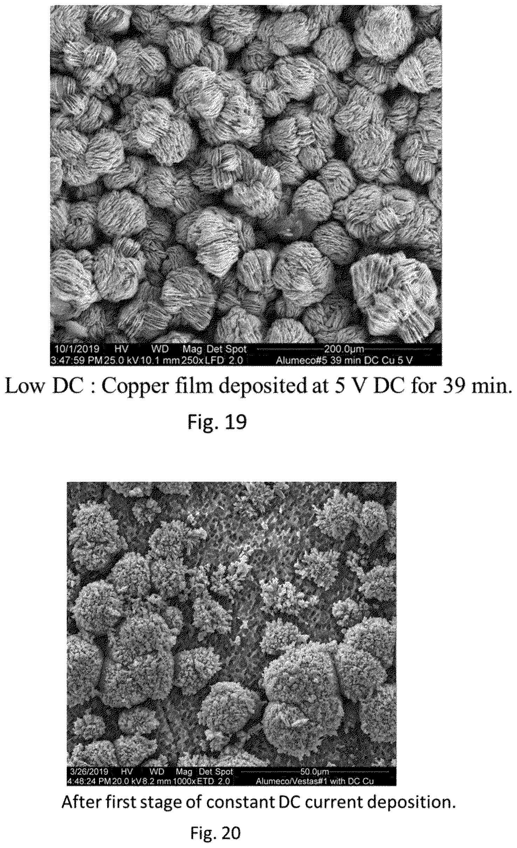

Experimenting with low voltages (below 10 V) gave some surprising results, as seen in fig. 19. In this case an alloy sample was used, and copper was deposited at 5 V for 39 min. It resulted in a film of micro-clusters of copper wires and lamellae.

In a further development, the inventor examined the effects of keeping the current constant during deposition, as the total current to be used for larger samples could be a critical issue for the industrialization. A sample of an alloy was treated at a current density of 0.125 A/cm2 for 6 min. The corresponding voltage was 8.2 V. An excess of loosely bound copper settling on the surface during the process was then brushed off the surface and the surface below the grown copper clusters became roughened, which indicated that etching of the surface oxide was taking place at this voltage, as seen in fig. 20.

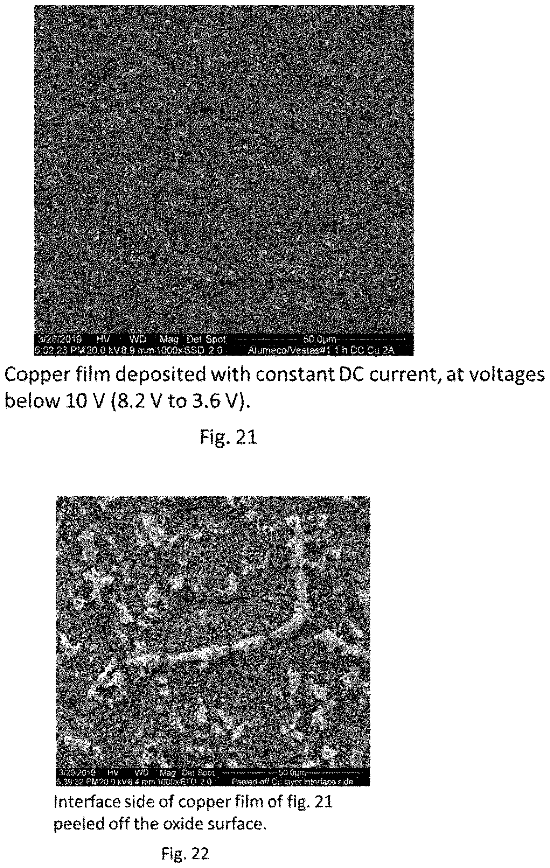

The process was then continued at a lower current density (0.05 A/ cm2) or a total current of 2 A, which happened for voltages from 4.1 V decreasing to 3.6 V. The overlayer structure resulting from this process is revealed by the SEM image shown in fig. 21, seeing the sample from above.

Thus, this procedure results in a microcrystalline, contiguous and mechanically stable film covering the surface completely. It can be treated mechanically by polishing and lapping, to give it a surface, which is smooth on a macroscopic scale.

This film could be lifted off the surface after dipping the sample in 70°C hot phosphoric acid for more than three hours, which created a slip between the film and the surface at the edges of the film. The whole film could then be peeled off as one piece, which could be studied to examine the interface between film and substrate. This interface of the film is imaged with the SEM, as shown in fig. 22. It shows that the film consists of regularly sized microcrystals and copper wires, which are pulled out of the pores. Thus, this film is clearly rooted in the pores, and its mechanical stability and attachment to the surface is secured in part by the liberation of copper wires from the surface by the etching away of the surface forming craters below the clusters.

Summarizing, this film has good mechanical, chemical and electrical properties, and may be polished to a macroscopically smooth finish. The electrical connection to the underlying aluminum substrate is of course somewhat limited by the presence of an insulating barrier oxide, but at a sufficiently high voltages across this oxide, conductivity should not be much hindered, similarly to the case for normal aluminum conductors, which are also covered by the natural oxide layer.

### Low voltage AC (not covered by the claims):

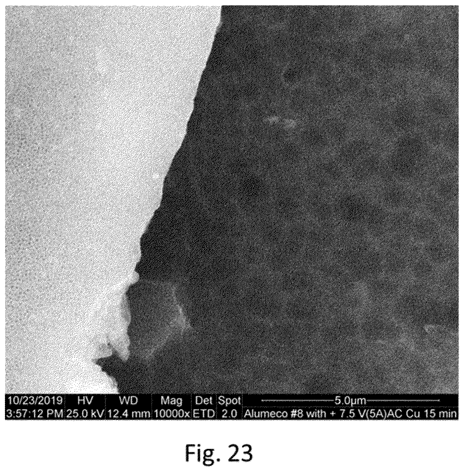

Pore filling and growth of copper films also occur with low voltage AC deposition, as shown in fig. 23. In this case 7.5 V(rms) AC was used for processing an alloy sample. The image shows how these conditions lead to etching of the surface, while the copper film (lighter gray at left in the image) growing on top of the surface with pores retains the pore structure like for the case shown for DC deposition at high voltage (cf. figs. 14 and 15). The film is more loosely bound than in the case of DC deposition, which is illustrated by the fact that part of the oxide films has fallen off the surface without etching or external mechanical influences. In other studies, it is verified that the pores become filled but that copper also in this case has a tendency to attach to the six apexes of the pore rims.

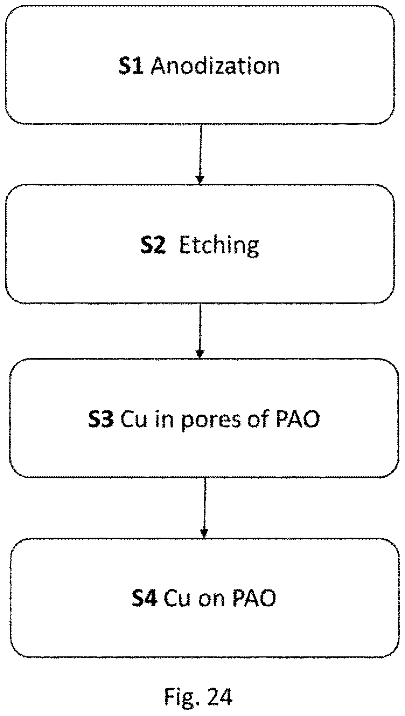

Figure 24 is a flow-chart of a method according to the invention; i.e. a process for for manufacturing a thin and stable copper 30 film on a porous aluminum oxide PAO film on an aluminum alloy substrate 10, cf. Fig. 1, the process comprising:
- **S1** anodization of the aluminum alloy substrate resulting in a regularly ordered PAO film; the PAO film and the remaining aluminum alloy thereby forming a PAO/aluminum substrate 50, cf. Fig. 2,
- **S2** optionally etching said PAO/aluminum substrate for further opening of the top of the pores 21 in the surface of said PAO film after said anodization, c.f. Fig. 2 (bottom), and
- electrodepositing copper on said PAO/aluminum substrate, the electrodepositing comprises the use of direct current (DC), preferably in the interval of 5-20 V, more preferably 10-15 V, so as to deposit metallic copper 30' and 30", cf. Fig. 4, both:
   1) **S3** in the open pores of the regularly ordered PAO film 21, c.f. Fig. 4, and simultaneously or subsequently,
   2) **S4** on the top of the regularly ordered PAO film 22,

so as to form a copper film 30 on top of the PAO film, cf. Figs. 5 and 11,
wherein said anodization is performed so as to improve the adhesion and electrochemical corrosion stability of the later electrodeposited copper on said PAO/aluminum substrate.

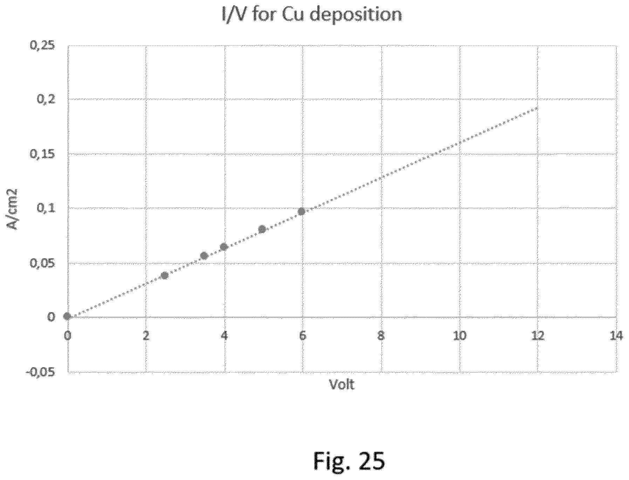

Fig. 25 shows a graph illustrating the measured relationship between the voltage and the current density at the beginning of the deposition process. The measurements were made for an aluminum block of a 6060 alloy, with approx. 10 micrometer deep pores. The interesting thing about the measurement (performed for 5 values of the voltage) is that it shows a linear relationship. In the figure, this connection is extended arbitrarily, to also be able to outline/extrapolate values for higher voltages.

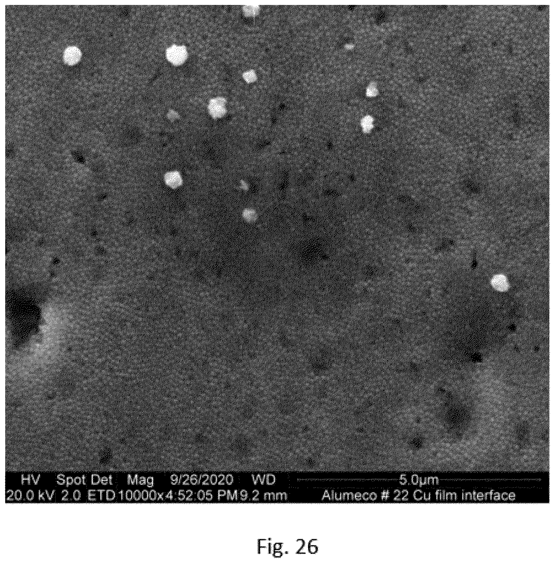

Fig. 26 shows an image of an aluminum substrate where the copper has been removed. The white dots are cupper residue, but the rest of the image shows the pores.

Formation of copper film by further deposition, in close contact with the copper in the pores, can be done by continuing the process after the pores are filled. It is preferable here for the structure of the film that it is only (initially) formed by copper, which grows out of the pores. Thereby, the film growth becomes of a self-organizing character, like what is described here:
As the copper grows out of the pores, it first forms some cohesive clusters on top of the pores, the size of which depends on the surface tension and interaction of the wires by van der Waals forces, and during the process, these clusters change the coverage of the surface, which is eventually completely covered by fused clusters. Subsequently, there is no significant growth on top of these clusters, whereby the process is self-controlling. The structure of the clusters depends on the voltage: at low voltages the clusters are covered by wire-shaped surfaces (at 5 V: clusters are about 50 micrometers in diameter), while at the highest voltages (diameter from 1 to 5 micrometers) formation of a more continuous film, with a delineation of the underlying pore structure. In these cases of higher voltages (above 10 V) during deposition and film growth, thicker films can be formed, as the growth of copper from the bath at longer deposition times now no longer appears to be coupled solely to the growth from the pores.

Finishing can take place in different ways. If a surface is desired, with filled pores, but which looks like normally anodized aluminum, after filling the pores, the surface can be polished mechanically or by using electropolishing, which etches a thin layer of the surface off. Thereby, the surface becomes shiny and any irregularities in the degree of coverage of the pore filling are removed. This gives the surface a maximum (for this type of surface structure of the copper in the pores) degree of antibacterial activity, and makes it easy to clean. This type of surface is also hydrophobic, some have called it superhydrophobic, so that it is "self-cleaning" when rinsing with clean water. For surfaces with an uneven copper film with roots in the pores, it will be advisable to polish it mechanically, which is easy as the copper film is soft, just as copper usually is, and it is firmly attached mechanically because it is anchored in the pores. This process can form a completely smooth and continuous thin copper film on the surface, which has a golden copper-colored skin and is clearly hydrophobic. Therefore, this type of finishing, with this type of film, will be maximally active as antibacterial protection of aluminum blanks, and looks aesthetically pleasing. For the more uniform films formed at higher voltages (and currents), because these films can be made thicker (up to 200 micrometers) by longer deposition, it will be possible to polish the film better to achieve a smoother finish on top of a larger thickness, which will be important for achieving a large contact surface and good conductivity in electrical and mechanical joints.

Regarding antibacterial properties; early in the process, experiments were performed with bacteria collected from toilet handles. These experiments showed that the bacterial activity on an anodized aluminum surface was very small and that a pure copper surface had 100% efficiency to kill bacteria after a certain period of time. During the same period, an anodized surface with partially filled pores had an effect that was between the other two surfaces.

In short, the invention relates to process for manufacturing a thin and stable copper film on a porous aluminum oxide (PAO) film on an aluminum alloy substrate. Initially, anodization of the aluminum alloy substrate results in a regularly ordered PAO film; and subsequently there is electrodeposited copper on this PAO/aluminum substrate, the electrodepositing comprises the use of direct current (DC), preferably 10-15 V, so as to deposit metallic copper both:
1) in the open pores of the regularly ordered PAO film and 2) on the top of the regularly ordered PAO film 22, so as to form a copper film 30 on top of the PAO film.

The anodization is performed so as to improve the adhesion and electrochemical corrosion stability i.e. with no, or little, galvanic corrosion of the electrodeposited copper on the PAO/aluminum substrate. The copper coverage is substantially continuous on the PAO/aluminum substrate.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

## Claims

1. A process for manufacturing a thin and stable copper (Cu, 30) film on a porous aluminum oxide (PAO, 20) film on an aluminum alloy substrate (Al, 10), the process comprising:
- anodization of the aluminum alloy substrate resulting in a regularly ordered PAO film; the PAO film and the remaining aluminum alloy thereby forming a PAO/aluminum substrate (50),
- optionally etching said PAO/aluminum substrate for further opening of the top of the pores (21) in the surface of said PAO film after said anodization, and
- electrodepositing copper on said PAO/aluminum substrate, the electrodepositing comprises the use of direct current (DC), preferably in the interval of 5-20 V, more preferably 10-15 V, so as to deposit metallic copper (30', 30") both:
1) in the open pores of the regularly ordered PAO film (21), and simultaneously or subsequently,
2) on the top of the regularly ordered PAO film (22),
so as to form a copper film (30) on top of the PAO film,
wherein said anodization is performed so as to improve the adhesion and electrochemical corrosion stability of the later electrodeposited copper on said PAO/aluminum substrate,
**characterised in that** the substrate - between and/or during any of the steps for electrodepositing copper 1) in the open pores of the substrate, and 2) on the top of the substrate so as to form a copper film on top of the substrate - is mechanically treated to remove, at least partly, any copper (30") electrodeposited on top of the substrate before the pores are filled with copper.

2. The process for manufacturing a copper film on a PAO/aluminum substrate according to claim 1, wherein a further anodization is performed to create a regularly ordered PAO film on the aluminum alloy substrate.

3. The process for manufacturing a stable copper film on a PAO/aluminum substrate according to claim 1, wherein the anodization comprises a gradual lowering of the anodization voltage from a first voltage level (V1) to a second voltage level (V2), the second voltage level being lower than the first voltage level, and wherein the second voltage level is kept substantially constant for a predefined period of time (T_V2) , wherein the first and the second voltage level, the predefined period of time (T_V2) and/or the ramping rate (dV/dt) between the first and the second voltage level is adjusted for controlling the thickness (d) of the PAO from the bottom of the pores down to the aluminum alloy substrate.

4. The process for manufacturing a copper film on a PAO/aluminum substrate according to claim 1, wherein electrodepositing copper on said substrate so as to deposit metallic copper 1) in the open pores of the substrate is performed at a pH below or around 3.

5. The process for manufacturing a copper film on a PAO/aluminum substrate according to claim 1, wherein electrodepositing copper on said substrate 2) to form a copper film on top of the substrate results in an average film thickness in the interval of 50-200 nm, preferably 75-150 nm.

6. The process for manufacturing a copper film on a PAO/aluminum substrate according to claim 1, wherein electrodepositing copper on said substrate is performed around 10-15 V, or higher, so as to deposit metallic copper 2) on the top of the substrate so as to form a substantially continuous copper film connected to the copper in the pores of the substrate.

7. The process for manufacturing a copper film on a PAO/aluminum substrate according to claim 1, wherein electrodepositing copper on said substrate is performed below 10 V and with a current density below 0.05 A/cm², so as to deposit metallic copper 2) on the top of the substrate so as to form a substantially continuous copper film.

8. The process for manufacturing a copper film on a PAO/aluminum substrate according to claim 1, wherein electrodepositing copper on said substrate is performed below 10 V and with a current density above 0.05 A/cm², so as to deposit metallic copper 2) on the top of the substrate so as to grow micro-cluster copper, the micro-clusters having groups of copper nano-wires and/or copper lamellae.

9. Use of the process for manufacturing a copper film on a PAO substrate according to claim 1 for growing nano-crystalline copper alloys on the PAO surface.

## Patentansprüche

1. Prozess zum Herstellen eines dünnen und stabilen Films aus Kupfer (Cu, 30) auf einem porösen Film aus Aluminiumoxid (PAO, 20) auf einem Aluminiumlegierungssubstrat (Al, 10), wobei der Prozess Folgendes umfasst:
- Anodisierung des Aluminiumlegierungssubstrats, was zu einem regelmäßig geordneten PAO-Film führt; wobei der PAO-Film und die verbleibende Aluminiumlegierung dadurch ein PAO/Aluminium-Substrat (50) bilden,
- gegebenenfalls Ätzen des PAO/Aluminium-Substrats zum weiteren Öffnen der Oberseite der Poren (21) in der Oberfläche des PAO-Films nach der Anodisierung,
und
- galvanisches Abscheiden von Kupfer auf das PAO/Aluminium-Substrat, wobei das galvanische Abscheiden die Verwendung von Gleichstrom (DC) umfasst, bevorzugt in dem Intervall von 5-20 V, bevorzugter 10-15 V, um metallisches Kupfer (30', 30") abzuscheiden, sowohl:
1) in den offenen Poren des regelmäßig geordneten PAO-Films (21), als auch
gleichzeitig oder anschließend
2) auf der Oberseite des regelmäßig geordneten PAO-Films (22), um einen Kupferfilm (30) auf der Oberseite des PAO-Films zu bilden,
wobei die Anodisierung durchgeführt wird, um die Adhäsion und elektrochemische Korrosionsstabilität des später galvanisch abgeschiedenen Kupfers auf dem PAO/Aluminium-Substrat zu verbessern,
**dadurch gekennzeichnet, dass** das Substrat - zwischen und/oder während eines beliebigen der Schritte zum galvanischen Abscheiden von Kupfer 1) in den offenen Poren des Substrats und 2) auf der Oberseite des Substrats, um einen Kupferfilm auf der Oberseite des Substrats zu bilden - mechanisch behandelt wird, um zumindest teilweise beliebiges Kupfer (30"), das auf der Oberseite des Substrats galvanisch abgeschieden ist, zu entfernen, bevor die Poren mit Kupfer gefüllt werden.

2. Prozess zum Herstellen eines Kupferfilms auf einem PAO/Aluminium-Substrat nach Anspruch 1, wobei eine weitere Anodisierung durchgeführt wird, um einen regelmäßig geordneten PAO-Film auf dem Aluminiumlegierungssubstrat zu erzeugen.

3. Prozess zum Herstellen eines stabilen Kupferfilms auf einem PAO/Aluminium-Substrat nach Anspruch 1, wobei die Anodisierung ein schrittweises Senken der Anodisierungsspannung von einem ersten Spannungspegel (V1) auf einen zweiten Spannungspegel (V2) umfasst, wobei der zweite Spannungspegel niedriger als der erste Spannungspegel ist, und wobei der zweite Spannungspegel für einen vordefinierten Zeitraum (T_V2) im Wesentlichen konstant gehalten wird, wobei der erste und der zweite Spannungspegel, der vordefinierte Zeitraum (T_V2) und/oder die Anstiegsrate (dV/dt) zwischen dem ersten und dem zweiten Spannungspegel eingestellt werden, um die Dicke (d) des PAO von der Unterseite der Poren bis hinunter zu dem Aluminiumlegierungssubstrat zu steuern.

4. Prozess zum Herstellen eines Kupferfilms auf einem PAO/Aluminium-Substrat nach Anspruch 1, wobei das galvanische Abscheiden von Kupfer auf das Substrat, um metallisches Kupfer 1) in die offenen Poren des Substrats abzuscheiden, bei einem pH-Wert unter oder um 3 durchgeführt wird.

5. Prozess zum Herstellen eines Kupferfilms auf einem PAO/Aluminium-Substrat nach Anspruch 1, wobei das galvanische Abscheiden von Kupfer auf das Substrat 2) zum Bilden eines Kupferfilms auf der Oberseite des Substrats zu einer durchschnittlichen Filmdicke in dem Intervall von 50-200 nm, bevorzugt 75-150 nm, führt.

6. Prozess zum Herstellen eines Kupferfilms auf einem PAO/Aluminium-Substrat nach Anspruch 1, wobei das galvanische Abscheiden von Kupfer auf das Substrat um 10-15 V oder höher durchgeführt wird, um metallisches Kupfer 2) auf die Oberseite des Substrats abzuscheiden, um einen im Wesentlichen durchgehenden Kupferfilm zu bilden, der mit dem Kupfer in den Poren des Substrats verbunden ist.

7. Prozess zum Herstellen eines Kupferfilms auf einem PAO/Aluminium-Substrat nach Anspruch 1, wobei das galvanische Abscheiden von Kupfer auf das Substrat unter 10 V und mit einer Stromdichte unter 0,05 A/cm² durchgeführt wird, um metallisches Kupfer 2) auf die Oberseite des Substrats abzuscheiden, um einen im Wesentlichen durchgehenden Kupferfilm zu bilden.

8. Prozess zum Herstellen eines Kupferfilms auf einem PAO/Aluminium-Substrat nach Anspruch 1, wobei das galvanische Abscheiden von Kupfer auf das Substrat unter 10 V und mit einer Stromdichte von über 0,05 A/cm² durchgeführt wird, um metallisches Kupfer 2) auf die Oberseite des Substrats abzuscheiden, um Mikrocluster-Kupfer zu züchten, wobei die Mikrocluster Gruppen von Kupfer-Nanodrähten und/oder Kupferlamellen aufweisen.

9. Verwendung des Prozesses zum Herstellen eines Kupferfilms auf einem PAO-Substrat nach Anspruch 1 zum Züchten nanokristalliner Kupferlegierungen auf der PAO-Oberfläche.

## Revendications

1. Procédé de fabrication d'un film de cuivre (Cu, 30) mince et stable sur un film d'oxyde d'aluminium poreux (PAO, 20) sur un substrat en alliage d'aluminium (Al, 10), le procédé comprenant :
- l'anodisation du substrat en alliage d'aluminium résultant en un film PAO ordonné de manière régulière ; le film PAO et l'alliage d'aluminium restant formant ainsi un substrat PAO/aluminium (50),
- éventuellement la gravure dudit substrat PAO/aluminium pour ouvrir davantage le haut des pores (21) dans la surface dudit film PAO après ladite anodisation,
et
- l'électrodéposition de cuivre sur ledit substrat PAO/aluminium, l'électrodéposition comprenant l'utilisation d'un courant continu (CC), de préférence dans l'intervalle de 5 à 20 V, plus préférablement de 10 à 15 V, de manière à déposer du cuivre métallique (30', 30 ") à la fois :
1. dans les pores ouverts du film PAO (21) ordonné de manière régulière , et simultanément ou ultérieurement,
2. au-dessus du film PAO (22) ordonné de manière régulière , de manière à former un film de cuivre (30) au-dessus du film PAO,
dans lequel ladite anodisation est réalisée de manière à améliorer l'adhésion et la stabilité à la corrosion électrochimique du cuivre électrodéposé ultérieurement sur ledit substrat PAO/aluminium,
**caractérisé en ce que**
le substrat - entre et/ou pendant l'une quelconque des étapes d'électrodéposition de cuivre 1) dans les pores ouverts du substrat et 2) au-dessus du substrat de manière à former un film de cuivre au-dessus du substrat - est traité mécaniquement pour éliminer, au moins en partie, tout cuivre (30") électrodéposé au-dessus du substrat avant que les pores ne soient remplis de cuivre.

2. Procédé de fabrication d'un film de cuivre sur un substrat de PAO/aluminium selon la revendication 1, dans lequel une anodisation supplémentaire est effectuée pour créer un film PAO ordonné de manière régulière sur le substrat en alliage d'aluminium.

3. Procédé de fabrication d'un film de cuivre stable sur un substrat de PAO/aluminium selon la revendication 1, dans lequel l'anodisation comprend un abaissement progressif de la tension d'anodisation depuis un premier niveau de tension (V1) jusqu'à un deuxième niveau de tension (V2), le deuxième niveau de tension étant inférieur au premier niveau de tension, et dans lequel le deuxième niveau de tension est maintenu sensiblement constant pendant une période de temps prédéfinie (T_V2), dans lequel le premier et le deuxième niveau de tension, la période de temps prédéfinie (T_V2) et/ou le taux de rampe (dV/dt) entre le premier et le deuxième niveau de tension est ajusté pour commander l'épaisseur (d) du PAO depuis le fond des pores jusqu'au substrat en alliage d'aluminium.

4. Procédé de fabrication d'un film de cuivre sur un substrat PAO/aluminium selon la revendication 1, dans lequel l'électrodéposition de cuivre sur ledit substrat de manière à déposer du cuivre métallique 1) dans les pores ouverts du substrat est réalisée à un pH inférieur ou proche de 3.

5. Procédé de fabrication d'un film de cuivre sur un substrat de PAO/aluminium selon la revendication 1, dans lequel l'électrodéposition de cuivre sur ledit substrat 2) pour former un film de cuivre au-dessus du substrat a pour résultat une épaisseur moyenne de film dans l'intervalle de 50 à 200 nm, de préférence de 75 à 150 nm.

6. Procédé de fabrication d'un film de cuivre sur un substrat de PAO/aluminium selon la revendication 1, dans lequel l'électrodéposition de cuivre sur ledit substrat est effectuée à environ 10 à 15 V, ou plus, de manière à déposer du cuivre métallique 2) au-dessus du substrat de manière à de manière à former un film de cuivre sensiblement continu relié au cuivre dans les pores du substrat.

7. Procédé de fabrication d'un film de cuivre sur un substrat PAO/aluminium selon la revendication 1, dans lequel l'électrodéposition de cuivre sur ledit substrat est réalisée en dessous de 10 V et avec une densité de courant inférieure à 0,05 A/cm², de manière à déposer du cuivre métallique 2) au-dessus du substrat de manière à former un film de cuivre sensiblement continu.

8. Procédé de fabrication d'un film de cuivre sur un substrat PAO/aluminium selon la revendication 1, dans lequel l'électrodéposition de cuivre sur ledit substrat est réalisée en dessous de 10 V et avec une densité de courant supérieure à 0,05 A/cm², de manière à déposer du cuivre métallique 2) au-dessus du substrat de manière à faire croître des micro-amas de cuivre, les micro-amas comportant des groupes de nanofils de cuivre et/ou de lamelles de cuivre.

9. Utilisation du procédé de fabrication d'un film de cuivre sur un substrat PAO selon la revendication 1 pour faire croître des alliages de cuivre nanocristallins sur la surface de PAO.
